# EUROPÄISCHE PATENTSCHRIFT

(11) **EP 0 621 795 B1**
(45) Veröffentlichungstag und Bekanntmachung des Hinweises auf die Patenterteilung: **03.06.1998**
(21) Anmeldenummer: 94900116.8
(22) Anmeldetag: 09.11.1993
(51) Int. Cl.: A61N 2/00

(54) **EINRICHTUNG ZUR BEEINFLUSSUNG VON ELEKTRISCHEN UND MAGNETISCHEN FELDERN NIEDRIGER FREQUENZ**
DEVICE FOR CONTROLLING LOW-FREQUENCY ELECTRIC AND MAGNETIC FIELDS
DISPOSITIF PERMETTANT D'INFLUER SUR DES CHAMPS ELECTRIQUES ET MAGNETIQUES DE BASSE FREQUENCE

(30) Priorität: 17.11.1992 DE 4238829
(43) Veröffentlichungstag der Anmeldung: 02.11.1994
(73) Patentinhaber: DR. FISCHER AG, FL-9490 Vaduz (LI)
(72) Erfinder: WARNKE, Ulrich, D-66133 Scheidt (DE)
(74) Vertreter: Engelhardt, Guido, Dipl.-Ing.
(86) Internationale Anmeldenummer: EP9303126
(87) Internationale Veröffentlichungsnummer: WO9411062

(56) Entgegenhaltungen:
- EP-A- 0 181 053
- EP-A- 0 217 011
- EP-A- 0 377 284
- WO-A-92/18873
- AT-B- 393 084
- US-A- 4 622 952
- US-A- 4 685 462
- Andrew A. Marino: Modern Bioelectricity, Marcel Dekker Inc., New York / Basel, 1988, Seiten 589-627

## Beschreibung

Die Erfindung betrifft eine Einrichtung zur Beeinflussung von lokalen elektrischen und magnetischen Feldern niedriger Frequenz, die auf eine in einem begrenzten Raumbereich befindliche leitfähige Struktur, wie die organische Substanz eines Lebewesens, einwirken. Solche Felder werden vor allem durch Hochspannungsleitungen, Bahnstromleitungen und Leitungen für die Stromversorgung in Häusern und daran geschlossene elektrische Geräte verursacht.

Wie seit längerem bekannt, haben elektrische und magnetische Felder niedriger Frequenz einen nicht unwesentlichen Einfluß auf organische Substanzen, und zwar über ihren Einfluß auf biologische Abläufe. Die Ursache liegt unter anderem in der Einwirkung auf Ionen in diesen leitfähigen Strukturen. Zur Vermeidung von Wiederholungen wird darauf bei der Erläuterung der Erfindung eingegangen. Dabei ist häufig nicht nur das Feld der Grundschwingung der Felder von Bedeutung, sondern auch von deren Oberwellen. In einer Spektralbetrachtung ergeben sich sozusagen Schwerpunktsfrequenzen, die durch die Grundschwingung und die Oberschwingungen bestimmt sind. Meist sind nur Oberschwingungen bis zur 3. Harmonischen von Interesse.

Der Erfindung liegt die Aufgabe zugrunde, in einem begrenzten Raumbereich die Wirkung solcher Felder zumindest wesentlich zu vermindern und zugleich gezielt eine Einwirkung auf eine Substanz zu ermöglichen.

Dies wird nach der Erfindung bei einer Einrichtung zur Beeinflussung von lokalen elektrischen und magnetischen Wechselfeldern niedriger Frequenz, die auf eine in einem begrenzten Raumbereich befindliche leitfähige Struktur, wie die organische Substanz eines Lebewesens, einwirken, bei der eine Aufnahmevorrichtung vorgesehen ist, die in dem begrenzten Raumbereich die Felder in Form von nach einem Koorodinatensystem, wie einem kartesischen System, orientierten Feldkomponenten aufnimmt, aus denen der Kompensation der Wechselfeder dienende Felder abgeleitet werden, dadurch erreicht, daß an die Aufnahmevorrichtung eine Meßvorrichtung angeschaltet ist, die der Bestimmung der von der Aufnahmevorrichtung aufgenommenen Feldkomponenten nach Amplitude, Frequenz und Orientierung dient, daß an die Meßvorrichtung über ein Steuerungsglied ein Sender angeschaltet ist, der über eine Vorrichtung in dem begrenzten Raumbereich ein Gegenfeld erzeugt, daß das Steuerungsglied, der Sender und die das Gegenfeld erzeugende Vorrichtung in Abhängigkeit von den Meßwerten der Meßvorrichtung so in der Frequenz, in der Stärke und der Koordinatenorientierung des Gegenfeldes einstellbar sind, daß das in dem begrenzten Raumbereich erzeugte Gegenfeld durch Interferenz mit den lokalen Feldern deren Wirkung auf die leitfähige Struktur zumindest näherungsweise kompensiert, und daß der Sender entweder über die an ihn angeschaltete Vorrichtung außer dem die lokalen Felder kompensierenden, in der Schwerpunktsfrequenz übereinstimmenden Gegenfeld ein weiteres Schwingungsfeld mit demgegenüber niedrigerer Frequenz, insbesondere mit einem Frequenzwert zwischen etwa 1 Hz und etwa 8 Hz oder etwa 10 Hz und etwa 30 Hz, in dem begrenzten Raumbereich erzeugt, oder in seiner Frequenz gegenüber der Schwerpunktsfrequenz der lokalen Felder um einen solchen Frequenzwert verschoben ist, daß in dem begrenzten Raumbereich eine Interferenzschwingung relativ niedriger Frequenz, bezogen auf die Schwerpunktsfrequenz dar lokalen Felder, insbesondere mit einem Frequenzwert zwischen etwa 1 Hz und etwa 8 Hz oder etwa 10 Hz und etwa 30 Hz entsteht.

Einrichtungen zur Kompensation störender Magnetfelder sind allgmein bekannt, so auch für Frequenzen des Stromversorgungsnetzes, beispielsweise durch die DE-OS 32 07 708 A1 und die DE-OS 32 09 453 A1. Diese Einrichtungen arbeiten hinsichtlich der Kompensation dreidimensional und verwenden u.a. Helmholtz-Spulen. Es fehlen aber die für die Erfindung charakteristischen Merkmale. Das gilt auch für die in der AT 393 084 B beschriebene Einrichtung zur Neutralisation von geologischen bzw. Störzoneneinflüssen. Die DE-OS 41 01 481 A1 und die PCT-Anmeldung Wo 92/18873 beschreiben die Kompensation von störenden magnetischen Feldern bei Geräten, die zur Messung der Kernresonanz bzw. der Elektronenspinresonanz dienen.

Von Vorteil ist es, wenn die Einrichtung mit einem die Auswertung auf die Feldkomponenten der lokalen Felder begrenzenden Frequenzfilter, insbesondere einem Tiefpaß, versehen ist, da hierdurch wirksam einer Selbsterregung der Einrichtung vorgebeugt werden kann. Das kann auch dadurch erreicht werden, daß eine Schleifenschaltung vorgesehen ist, die in bestimmten zeitlichen Abständen für eine kurze Zeitspanne die Einstellung des Steuerungsgliedes nach vorgegebanen Frequenzkriterien überprüft und bei Erfüllung derselben jeweils den Sender von den Aufnahmevorrichtungen entkoppelt.

In Weiterbildung der Erfindung ist eine zweite Aufnahmevorrichtung vorgesehen, die der Bestimmung des in dem begrenzten Raumbereich herrschinden magnetischen Gleichfeldes, insbesondere des Erdmagnetfeldes nach Stärke und Koordinatenausrichtung vorgesehen ist, und außerdem eine Vorrichtung vorgesehen, der die Signale der ersten und zweiten Aufnahmevorrichtung zugeführt werden und die der Bestimmung von in der leitfähigen Struktur auftretenden Resonanzen dient.

Von Vorteil ist es auch, eine erfindungsgemäße Einrichtung in der Weise auszubilden, daß die Frequenz der Interferenzschwingung und die Sendeenergie in Abhängigkeit von dem Erdmagnetfeld so hoch einstellbar sind, daß die Interferenzschwingung im Zusammenwirken mit dem magnetischen Gleichfeld die Bedingungen für eine Zyklotronresonanz mit Ionen, insbesondere Kalzium-Ionen (Ca⁺⁺), von Kalium-Ionen (K⁺) oder von Natrium-Ionen (Na⁺), oder einer nuclear-magnetic-resonance (NMR) einer organischen Substanz erfüllt.

Bewährt hat es sich, die Einrichtung mit einer die Feldkomponenten kartesisch erfassenden Aufnahmevorrichtung zu versehen und in der Weise auszubilden, daß für jede Koordinatenrichtung je ein Aufnehmer für die elektrische und ein Aufnehmer für die magnetische Feldkomponente vorgesehen ist und diese Aufnehmer an die Eingänge eines Multiplexers angeschaltet sind und daß der Multiplexer-Ausgang vorzugsweise über einen den auszuwertenden Frequenzbereich aussiebenden Filterzweig, wie einen Tiefpaß, mit der Meßvorrichtung verbunden ist. Zweckmäßig sind an den Multiplex-Ausgang mehrere Filterzweige für unterschiedliche Frequenzbereiche angeschaltet. An jeden dieser Filterzweige kann dann eine Meßvorrichtung angeschaltet sein. Die Meßeinrichtung besteht vorzugsweise aus einer Tracking-Filter-Schaltung in PLL-Ausführung, die einen Schaltungsteil für die Abgabe eines der zu ermittelnden Frequenz entsprechenden Signals und einen Schaltungsteil zur Abgabe eines der zu ermittelnden Amplitude entsprechenden Signals umfaßt. Die Ausführung mit einem Multiplexer erlaubt es in einfacher Weise, am Multiplexer einen weiteren Eingang für an der organischen Substanz kapazitiv influenzierte elektrische Felder vorzusehen. Auch ein weiterer Eingang für in der organischen Substanz entstehende Felder ist auf diese Weise mittels des Multiplexers einfach realisierbar.

Zweckmäßig werden die Ausgänge der einzelnen Meßeinrichtungen mit entsprechenden Eingängen eines weiteren Multiplexers verbunden, an dessen Ausgang ein vorzugsweise digital arbeitender Mikrokontroller angeschaltet ist, mit dem Einstellorgane für die einzelnen Feldkomponenten des in dem begrenzten Raumbereich von der Einrichtung erzeugten Feldes gesteuert werden.

Als Aufnahmevorrichtung für kapazitiv auf die leitfähige Struktur einwirkende oder in dieser entstehende elektrische Felder haben sich an die Struktur anlegbare galvanische Elektroden als zweckmäßig erwiesen. Man kann über solche Elektroden beispielsweise von der organischen Struktur eines Lebewesens einem Elektroenzephalogramm (EEG) entsprechende Signale ableiten und mit diesen ein Einstellkriterium für Frequenz und Amplitude der vorerwähnten Interferenzschwigung gewinnen. Als Aufnahmevorrichtung für induktiv auf die leitfähige Struktur einwirkende magnetische Feldar empfehlen sich Spulen und als Aufnahmavorrichtung für das in dem begrenzten Raumbereich einwirkende magnetische Gleichfeld, wie das Erdmagnetfeld empfiehlt sich die Verwendung eines dreidimensional aufnehmenden Magnetoflux-Meters oder eines vorzugsweise temperaturkompensierten Hallgenerators.

Nachstehend wird die Erfindung anhand der Ausführungsbeispiele wiedergebenden Zeichnung näher erläutert. In der Zeichnung zeigen:
- Figur 1: eine Prinzipschaltung einer erfindungsgemäßen Einrichtung für eine Störfeldauslöschung in einem Raum, beispielsweise einem Wohnraum,
- Figur 2: eine Prinzipschaltung einer erfindungsgemäßen Einrichtung für die Induzierung eines Schwingungsfeldes, beispielsweise in einem Schlafraum, mit dem Zweck einer Schlafförderung,
- Figur 3: eine Prinzipschaltung einer weiteren erfindungsgemäßen Einrichtung eines Schwingungsfeldes, beispielsweise in einem Schlafraum, mit dem Zweck einer Schlafförderung,
- Figur 4: eine Prinzipschaltung für die Aufnahme der in einer organischen Substanz auftretenden oder ausgelösten Felder, die auch für die Aufnahme der in einer organischen Substanz kapazitiv influenzierten Ladung, die an der Oberfläche der Substanz auftritt, geeignet ist, sowie induzierte Felder meßbar macht,
- Figur 5: ein Ausführungsbeispiel für die Kompensation des 50 Hz-Wechselfeldes wie es in Wohnungen häufig auftritt,
- Figur 6: ein Diagramm zur Veranschaulichung der bekannten Beziehung zwischen der magnetischen Flußdichte, gemessen in Gauß und der Frequenz in Hertz, bei der eine Ionenresonanz oder eine NMR auftritt, und zwar für Kalium-, Natrium- und Kalzium-Ionen,
- Figur 7: eine in der Übertragungstechnik bekannte Schaltung zur dispersionsfreien Phasenschiebung, wie sie auch für den Zweck einer Signalinvertierung bei erfindungsgemäßen Einrichtungen anwendbar ist.
- Figur 8: ein weiteres Ausführungsbeispiel der Erfindung für die Feststellung der magnetischen Feldkomponenten eines im begrenzten Raumbereich vorhandenen magnetischen Gleichfeldes, eines magnetischen Wechselfeldes, induzierter elektrischer Felder und in einer in dem Raum befindlichen organischen Substanz entstehender oder darin ausgelöster Felder
und
- Figur 9: eine Übersicht der in der organischen Substanz eines Menschen auftretenden Felder.

Da die Erfindung und die erfindungsgemäßen Einrichtungen vornehmlich Geräte betreffen, die im Zusammenhang mit der Beeinflussung einer organischen Substanz durch elektrische und magnetische Felder stehen, wird zunächst auf Fakten eingegangen, aus denen sich die Zusammenhänge erkennen lassen.

Beispielsweise ist der Körper eines Lebewesens in erster Näherung als ein mit Elektrolyt gefüllter Raum anzusehen. Das gilt ganz besonders für den Kopfbereich, der im Grunde genommen eine mit Elektrolyt gefüllte Kugel ist. Damit stellt dieser Raum, vor allem der Kopfbereich, eine Art Kugelantenne für elektrische und magnetische Felder dar. Im Gehirnelektrolyten des Kopfbereiches liegt ein Geflecht von Nervenzellen und Ausläufern (Neuronen). Befinden sich diese Nervenzellen in aktiver Kommunkikation, so werden sie selber zu Sendern elektrischer und magnetischer Felder. Die Felder der einzelnen interferieren und addieren sich zu Summenfeldern, die beispielsweise als EEG (Elektroenzephalogramm) elektrisch in der Kopfregion abgegriffen und aufgezeichnet werden können. Erreichen vagabundierende lokale Außenfelder um den Kopfbereich herum eine kritische Größe, so interferieren sie mit den im Gehirn erzeugten Feldern. Dabei kann eine Amplituden- und/oder Frequenzmodulation der im Gehirn erzeugten Felder eintreten, die biologische Rückwirkungen auslöst. Die kritische Größe solcher störender Außenfelder, bei der das festgestellt wurde, beginnt nach dem Stand der derzeitigen Feststellungen für magnetische Wechselfelder bei etwa 0,4 Mikrotesla und für elektrische Wechselfelder bei etwa 5 Volt/Meter. Kapazitiv eingekoppelte Felder wirken sich in der Tiefe des Gehirns eines Lebewesens vor allem als Verschiebungs- und Ausgleichsströme im Elektrolyten aus, während induktiv eingekoppelte und eingeprägte Felder praktisch in jeder Schichttiefe des Gehirns das Summenfeld verändern können. Sowohl der kapazitive als auch der induktive Anteil solcher lokaler Felder kann biologische Folgeerscheinungen auslösen. In mehreren Untersuchungen konnte festgestellt werden, daß bestimmten Hormonen, wie dem Melatonin, denen für den Schlaf und damit die Regeneration eines Lebewesens und beispielsweise für die Abwehr von Krebserkrankungen, eine hohe Bedeutung zukommt, unter dem Einfluß äußerer Störfelder nachts nicht mehr ausreichend gebildet werden. Weiterhin wurde festgestellt, daß die innerhalb des Gehirns gelegene Epiphyse (syn. Zirbeldrüse; Pinealorgan), die innerhalb des Gehirns normalerweise Melatonin und zwei weitere Hormone herstellt, außerordentlich sensibel auf das magnetische Erdfeld und ähnlich schwache magnetische Gleichfelder ist. Die magnetischen Gleichfelder steuern im Zusammenwirken mit den elektrischen Wechselfeldern die Aktivitäten von Enzymen (SNAT = Serotonin-N-Acetyl-Transferase und HIOMT = Hydroxyindol-O-Methyl-Transferase) in der Epiphyse. Beide Enzyme reagieren auf elektrische und magnetische Felder und spezifische Frequenzen derselben. Der Serotonin-Pegel ist u.a. abhängig davon, wieviel Tryptopan als Vorstufe von Serotonin aus der Nahrung ins Blut gelangt und von diesem in das Gehirn transportiert wird. Serotonin ist für das psychische Wohlbefinden eines Lebewesens, vor allem des Menschen wesentlich mitverantwortlich. Wenn - tagsüber - viel Licht in der Umgebung eines Lebewesens ist, ergibt sich ein höherer Serotoningehalt im Gehirn. Dieses Serotonin wird nachts in Melatonin umgewandelt, wobei gegen zwei Uhr morgens der Melatonin-Spiegel am höchsten ist. Melatonin vermittelt dann die Bildung von Vasotonin, das den erholsamen Schlaf aufbaut. Wesentliches Zentrum für diesen ganzen Hormon-Umwandlungsprozeß und den Vermittlungsprozeß ist damit die Epiphyse, die ein kleiner Gehirnanhang im Gehirnzentrum ist.

Die Epiphyse kann offensichtlich magnetische Felder geringster Stärke "anzapfen" und ihnen Informationen entnehmen. Bei Tieren, wie Fischen, Lurchen und Vögeln ist dieser Vorgang gut dokumentiert. Daß Felder relativ minimaler Stärke bereits zur Organismus-Beeinflussung ausreichen, beruht darauf, daß elektromagnetische Resonanzen, wie die Elektronenspinresonanz und die magnetische Kernresonsnz auslösend wirken. Alle derartigen Resonanzen setzen voraus, daß auf ein Atom, ein Elektron oder auf ein Molekül ein stationäres Magnetfeld (Erdfeld oder Magnetfeld von Eisenteilen, Heizungsröhren usw.) zusammen mit einem elektrischen bzw. magnetischen Feld spezifischer Frequenz einwirken. Für den Menschen sind in dieser Hinsicht besonders resonenzempfindlich die Kalzium-Ionen (Ca⁺⁺), die Natrium-Ionen (Na⁺) und die Kalium-Ionen (K⁺), also gerade die wichtigsten Zellionen.

Wie bereits erwähnt, werden die zur Resonanz führenden, schwingenden elektrischen Felder, die zum Erdfeld passen, im Organismus selbst produziert, und zwar hauptsächlich von Nervenzellen. Vor allem vom Gehirn als Organ, das fast ausschließlich aus Nervenzellen besteht, ist dies seit langem bekannt und wird als ausstrahlendes elektrisches Feld (EEG) bzw. als ausstrahlendes magnetisches Feld (MEG) gemessen. Wenn tagsüber ein Lebewesen wie der Mensch aktiv ist, nimmt dieses Gehirnfeld als aufaddierte Summe aller aktiven Nervenzellen Frequenzen um 20 bis 25 Hertz an. Nachts, im Tiefschlaf schwingt es mit Frequenzen weit unter 10 Hertz oftmals bis hinunter zu Werten um 3 Hertz. Wie weitere Festellungen ergaben, liegen diese Frequenzen zusammen mit dem natürlichen Erdfeld tagsüber im Frequenzbereich von Zyklotonresonanzen, während die nachts erzeugten niedrigeren Frequenzen des Gehirns (ca. 3 Hz bis ca. 7 Hz) normalerweise keine Resonanzen bewirken.

Ein EEG, das bekanntlich als unipolare oder bipolare Ableitung der Potentialschwankungen von der Kopfhaut abgenommen wird, läßt bei einer Analyse seines Verlaufs nach der Ablaufschnelligkeit klassifizierbare Wellen erkennen, und zwar:

| | | |
|---|---|---|
| Alpha-Wellen = | 9 bis 12 (13) | Schwankungen pro Sek. |
| Beta- Wellen = | 14 bis 30 (50) | dto. |
| Delta-Wellen = | 0,5 bis 3,5 | dto. |
| Theta-Wellen = | 4 bis 7 | dto. |

bei einer Spannungshöhe zwischen 10 und 100 Mikrovolt, wobei mit zunehmender Verlangsamung die Spannungshöhe der Wellen zunimmt.

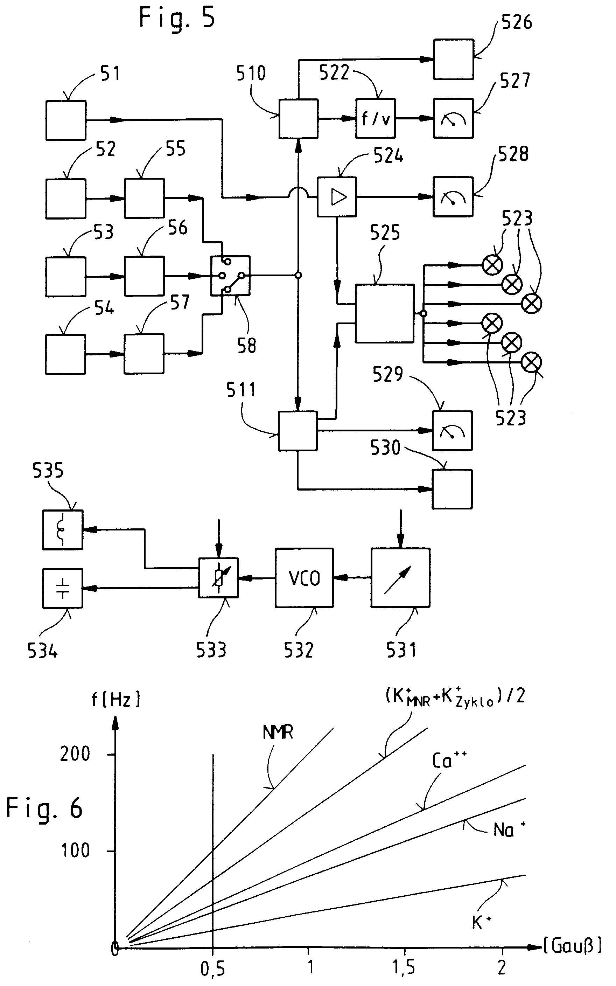

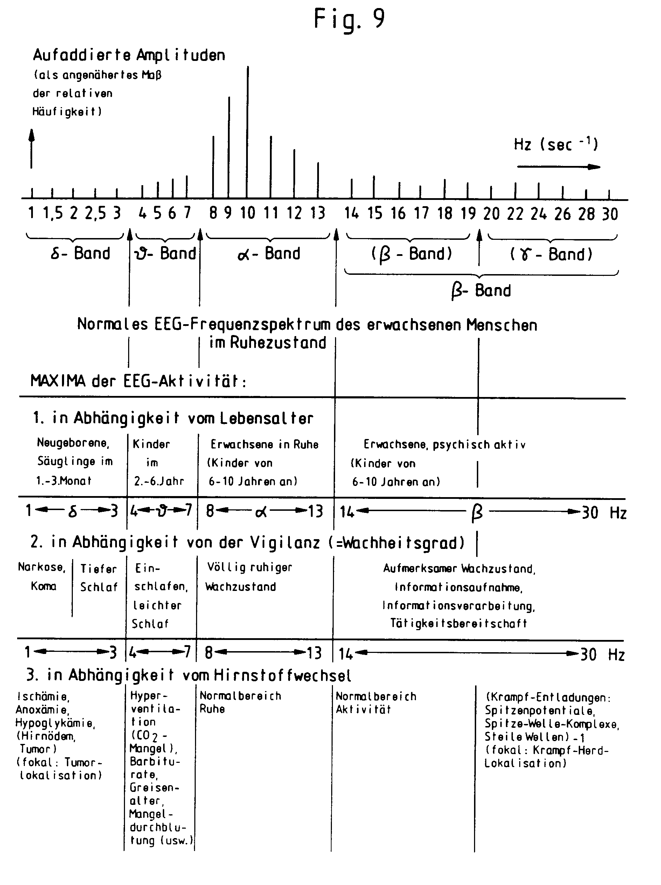

Eine Übersicht über durch Messung ermittelte Resonanzen vom Zyklotron-Typ und vom NMR-Typ gibt die Figur 6, während die Figur 9 eine Übersicht des EEG-Spektrums eines normalen Menschen nach ungefährer Amplitude und zugegehöriger Frequenz zeigt.

Das in der Nacht normalerweise auftretende Ausbleiben der Resonanzen und die damit verbundene lebenswichtige Hormonausschüttung kann jedoch durch lokale Felder gestört werden. Eine Ursache ist eine solche Verzerrung des wirksamen magnetischen Gleichfeldes, wie des Erdfeldes, daß vor allem im Gehirnbereich höhere oder auch niedrigere lokale Felder auftreten bzw. ausgebildet sind. Dies ist vor allem durch ferromagnetische Metalle, z. B. von den Federungs-Spiralen eines Bettes, von Metallregalen, Heizungskörpern und mit Gleichstrom betriebenen Geräten möglich. Weiterhin führen Felder von Stromversorgungsanlagen zu vagabundierenden magnetischen Feldern, die im Gehirnbereich elektromotorische Kräfte induzieren, die in der Größenordnung der natürlichen Felder des Gehirns und darüber liegen können. Dies gilt auch für das Umgebungsfeld von Hochspannungsleitungen und praktisch alle elektrischen Geräte, vor allem des Haushalts.

Es kommt also entscheidend darauf an, entweder die lokalen Felder überhaupt in ihrer Wirkung in dem begrenzten Raumbereich zu kompensieren und/oder durch Felder bzw. Schwingungen in den bereits genannten Frequenzbereichen (etwa 3 Hz bis etwa 8 Hz und etwa 10 Hz bis etwa 30 Hz) die natürlichen Felder zu unterstützen oder zu bilden.

Die nachstehend anhand von Ausführungsbeispielen näher beschriebenen erfindungsgemäßen Einrichtungen bieten hierfür eine Möglichkeit.

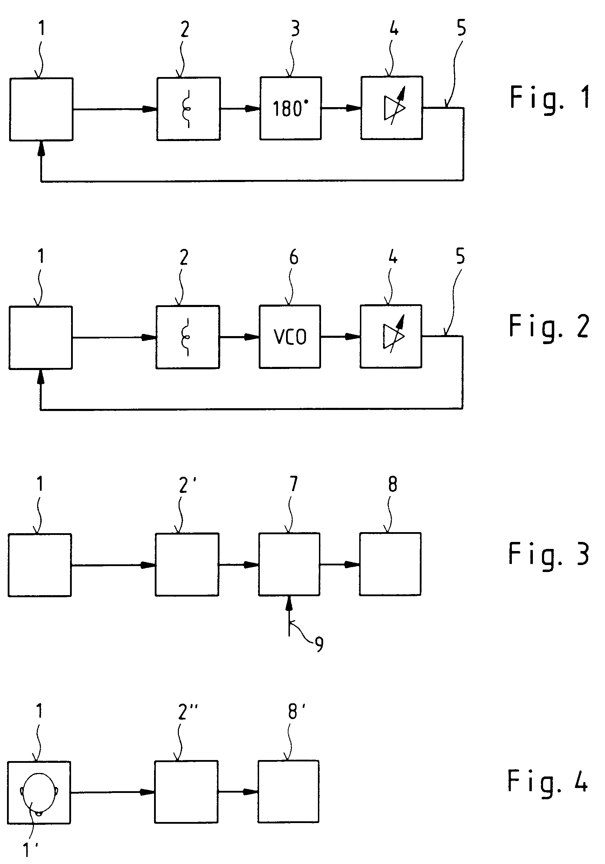

In der Figur 1 ist mit 1 ein begrenzter Raumbereich, z. B. ein Wohnraum bezeichnet, in dem von den Stromversorgungsleitungen ein magnetisches 50 Hz-Schwingungsfeld erzeugt wird. Dieses Schwingungsfeld kann auch Oberwellen enthalten. Dieses Feld wird mittels einer Aufnahmevorrichtung 2, die aus Spulen besteht, in Form von Feldkomponenten aufgenommen, die nach einem kartesischen Koordinatensystem (x, y, z) orientiert sind. Das x-, das y- und das z-Signal werden in dem Aufnehmer 2, bzw. einer mit diesem verbundenen Meßschaltung nach Amplitude und Frequenz bestimmt, in einem Inverter 3 in der Phase umgekehrt und über einen in der Amplitude seiner Ausgangssignale regelbaren Verstärker 4, der als Sender für die einzelnen Feldkomponenten fungiert, und nicht näher dargestellte Spulen, getrennt nach den Kordinaten in den begrenzten Reumbereich zurückgestrahlt. Die Amplituden der vom Sender erzeugten Feldkomponenten sind dabei ebenso wie die Phasen so eingestellt, daß das ursprüngliche 50 Hz-Feld fast völlig kompensiert wird. Die Einstellung des Senders 4 hinsichtlich der Amplituden der Feldkomponenten kann ebenso wie die Phaseneinstellung manuell oder mittels einer Regelschaltung selbsttätig erfolgen.

In der Figur 2 ist das Prinzip einer erfindungsgemäßen Einrichtung gezeigt, bei der die von einem Sender 6 in den begrenzten Raumbereich abgestrahlten Feldkomponenten (x, y, z) in der Frequenz um einen niedrigen Frequenzwert verschieden sind. Herrscht in 1 beispielsweise ein lokales 50 Hz-Feld, so haben beispielsweise die abgestrahlten Feldkomponenten eine Frequenz von 46 Hz. Es entsteht dadurch im Raum 1 eine pulsierende Schwebung mit einer Frequenz von 4 Hz, neben den sich periodisch kompensierenden 50 Hz-Schwingungen und 46 Hz-Schwingungen. Die Schwebung bzw. Interferenzschwingung kann für eine gezielte Beeinflussung der leitenden Substanz in dem begrenzten Raumbereich genutzt werden. Diese Ausführung ist vor allem auch für einen Schlafraum gedacht, weil durch die Interferenzschwingung die Schlafeinleitung und der Schlafzustand günstig beeinflußbar sind.

Eine vorteilhafte Abwandlung des Prinzips nach der Figur 2 besteht darin, daß die über die Spulenaufnehmer 2 erfaßten Feldkomponenten in einer Überlagerungsstufe mit der Schwingung des örtlichen Senders 6, der z. B. in an sich bekannter Weise als VCO (Voltage Controlled Oscillator) ausgebildet ist, gemischt werden und die hierbei entstehende, frequenztiefere Seitenbandschwingung über eine Tiefpaßschaltung zusammen mit einer das lokale Feld kompensierenden Schwingung entsprechend Figur 1 in den begrenzten Raumbereich eingestrahlt wird.

Eine Prüfung auf Zyklotron- und NMR-Resonanzen kann, wie Figur 3 im Prinzipschaltbild zeigt, dadurch erfolgen, daß mit einem Magnetoflux-Aufnehmer oder Hallgenerator 2', das stationäre magnetische Gleichfeld in dem begrenzten Raumbereich 1 in Form von Feldkomponenten gemessen und mit den über den Eingang 9 zugeführten, nach den vorstehenden Beispielen aufgenommenen und/oder erzeugten Wechselfeldkomponenten in einem Rechner 7 ausgewertet und das Ergebnis in einer Anzeigevorrichtung 8 erkennbar gemacht wird.

Signale, die am Körper außen, z. B. durch kapazitive Einstreuung auftreten, können gemäß Figur 4 über galvanische Elektroden-Aufnehmer 2'', die an der organischen Substanz anliegen, gemessen werden. Das gilt auch für die in der organischen Substanz erzeugten bzw. verursachten Felder, die sich durch das Anlegen der Elektroden 2 an die Kopfhaut 1' als eine Art EEG messen lassen.

Bei der Einrichtung nach der Figur 5 ist ein Hallgenerator oder ein Magnetoflux-Meter 51 als Aufnehmer für das stationäre magnetische Feld, eine galvanische Hand-Elektrode 52 als Aufnehmer für das auf einen nicht dargestellten Körper kapazitiv induzierte elektrische lokale Wechselfeld, eine Luftspule 53 - auch hierfür ist stattdessen ein Magnetoflux-Meter einsetzbar - als Aufnehmer für das auf den Körper im begrenzten Raumbereich einwirkende lokale magnetische Feld und eine galvanische Elektrodenanordnung 54 als Aufnehmer der Gehirnströme des Körpers dargestellt. Die Aufnehmer 51 und 52 nehmen das jeweilige Feld dreidimensional auf. Aus Gründen der Übersichtlichkeit zeigt die Figur 5 nur die Schaltung für jeweils eine der mit jedem der Aufnehmer erhaltenen drei raumorientierten Feldkomponenten. Die Signale der Aufnehmer 52, 53 und 54 werden über Verstärker den Eingängen eines Multiplexers 58 zugeführt, der zur seriellen Verarbeitung die Signale an zwei PLL-Schaltungen 510 und 511 abgibt. Die beiden PLL-Schaltungen sind von an sich bekannter Art und dienen als jeweils in Verbindung mit einem Multiplizierer als Tracking-Filter zur Messung von Amplitude und Frequenz der über die Aufnehmer 52, 53 und 54 aufgenommenen Signale. Die PLL-Schaltung 510 (522 und 526) bestimmt den Frequenzwert und bringt diesen über einen Frequenz/Spannungs-Wandler 522 zu einer Anzeige 527. Die lock-in-Kontrolle (Einrastkontrolle) erfolgt in dem Schaltungsteil 526. Analog hierzu bringt die PLL-Schaltung 511 (530) den zugehörigen Amplitudenwert zur Anzeigevorrichtung 529. Der Baustein 530 ist die zugehörige lock-in-Kontrolle. Die einschlägige PLL-Technik ist beispielsweise in dem Buch "Einführung in die PLL-Technik" von Geschwinde, erschienen im Vieweg-Verlag, Braunschweig, 1978, u. a. in den Kapiteln 2.2.1 und 2.2.2. ausführlich beschrieben, so daß von einer näheren Behandlung abgesehen werden kann.

Von der PLL-Schaltung 511, die der Amplitudenmessung dient, wird ferner ein Komparator 525 gespeist, dessen zweitem Eingang über einen Verstärker 524 mit Tiefpaßverhalten das vestärkte Ausgangssignal des Hallgenerators 51 zugeführt wird. Das Ausgangssignal des Verstärkers wird außerdem einer Anzeige 528 zur exakten Amplitudenwertbestimmung des über 51 aufgenommenen stationären magnetischen Feldes im begrenzten Raumbereich zugeführt. Der Komparator 525 setzt die beiden ihm zugeführten Signale zueinander in Relation und veranlaßt bei Auftreten von Werten, die einer Zyklotron-Resonanz und/oder einer NMR-Resonanz entsprechen, das Aufleuchten der entsprechenden Leuchtdiode in einem Leuchtdioden-Array 523. Die in der Zeichnung oberen drei Leuchtdioden sind für die Zyklotron-Resonanzen der Ionen von Kalzium, Natrium und Kalium vorgesehen und die unteren drei Leuchtdioden für NMR-Resonanzen der drei Elemente.

Ein VCO 532 erzeugt die für die Bildung des Gegenfeldes benötigte Schwingung, die über einen Amplitudenregler in Form eines regelbaren Dämpfungsgliedes einer ein magnetisches Wechselfeld im begrenzten Raumbereich erzeugenden Spule 535 und einer ein elektrisches Wechselfeld, entsprechend dem anhand der Figuren 1 und 2 erläuterten Prinzip, im begrenzten Raumbereich aufbauenden kapazitiven oder galvanischen Elektrode zugeführt wird. Auch hier ist nur die Schaltung für jeweils eine der drei Feldkomponenten der beiden Felder dargestellt. An sich könnte die Frequenz-/Phasen- und Amplitudeneinstellung des Senders über entsprechende, an sich bekannte Einstellorgane manuell erfolgen. Praktischer ist jedoch eine selbsttätige Einstellung. So kann die Frequenz des VCO 533 in einem Vergleicher 531 mit der Frequenz des lokalen Feldes in Relation gebracht werden und das daraus gewonnene, ein Maß für einen eventuellen Frequenzunterschied darstellende, Signal in an sich bekannter Weise als Regelgröße für den VCO verwendet werden. Ebenso kann der mit der entsprechenden PLL-Schaltung erhaltene Amplitudenwert der lokalen Felder zur Einstellung des Amplitudenreglers dienen.

Der VCO kann entweder exakt auf die Schwerpunktsfrequenz des lokalen Wechselfeldes abgestimmt werden, oder zur Bildung der bereits anhand der Figur 2 erläuterten Interferenzschwingung um einen vorgegebenen Frequenzwert verschieden von der Schwerpunktsfrequenz des lokalen Wechselfeldes eingestellt werden. Auch kann der VCO 532 durch eine Generatorschaltung ersetzt werden, der entsprechend der anhand der Figur 2 erläuterten Alternative eine exakt kompensierende Schwingung und eine zusätzliche Schwingung erzeugt, die als Ersatz für die dort erläuterte Interferenzschwingung dient.

Die Figur 6 zeigt in einem Diegramm den Zusammenhang zwischen den Resonanzen (in Hertz) der Ionen von Kalzium, Natrium und Kalium und der magnetischen Flußdichte (in Gauß). Die drei mit den Elementen Ca. Na und K bezeichneten Verläufe gelten für die Zyklotron-Resonanzen der Ionen dieser Elemente. Die anderen gelten für eine reine K⁺-NMR-Resonanz und eine Misch-Resonanz.

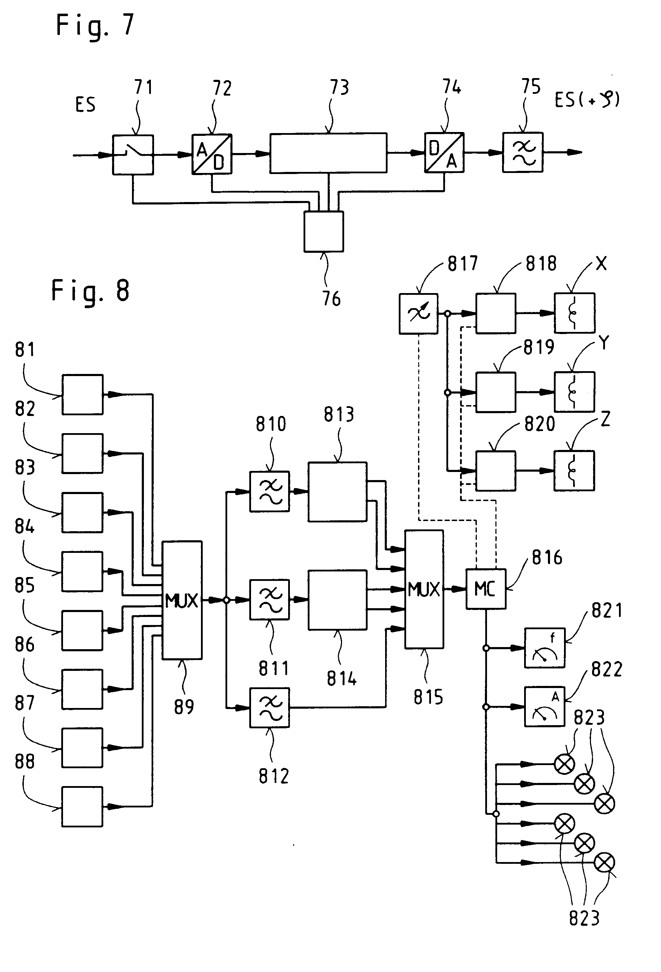

Die in der Figur 7 schematisch gezeigte Phasenschieber-Schaltung arbeitet auf digitaler Basis und ist seit etwa 1970 bekannt und wird beispielsweise in Speicherosilloskopen zur dispersionsfreien Verzögerung von Signalen angewendet. Das zu verzögernde Signal ES wird mittels eines Samplers 71 abgetastet. Die einzelnen Amplitudenproben der Abtastfolge werden in einem Analog-Digital-Wandler 72 in eine Folge von PCM-Signalen umgesetzt, die dann eine digital arbeitende Laufzeitkette 73 durchlaufen. Am Ausgang der Laufzeitkette 73 wird die Folge von PCM-Signalen mittels eines Digital-Analog-Wandlers 74 wieder in eine Folge von Amplitudenproben umgewandelt, aus der durch Umformung in einem der Unterdrückung unerwünschter Frequenzen dienenden Tiefpaß 75 das ursprüngliche Signal, jedoch zeitverzögert als Kontinuum entnommen werden kann. Die Zeitverzögerung wird durch die Taktfrequenz des Taktgeneretors 76 und die Anzahl der in Übertragungsrichtung hintereinanderliegenden Speicherzellen der Laufzeitkette 73 bestimmt. Bei einer vereinfachten Ausführungsform können auch die beiden Wandler 72 und 75 entfallen und die Laufzeitkette als Kette aus Ladungstransfer-Gliedern vom CCD-Typ ausgebildet werden. Auch eine "bucket-bridge-delay"-Schaltung, oder eine sogenannte "Eimerkette-Schaltung" sind ebenso anwendbar wie ein "analoger Phasenschieber" in Form eines Allpasses.

Bei dem Ausführungsbeispiel nach der Figur 8 sind die Bausteine 81 bis 88 Aufnehmer gemäß folgender Zuordnung:
- 81 Aufnehmer: für die x-Komponente des Erdfeldes
- 82 Aufnehmer: für die y-Komponente des Erdfeldes
- 83 Aufnehmer: für die z-Komponente des Erdfeldes
- 84 Aufnehmer: für die x-Komponente des lokalen magnetischen Wechselfeldes
- 85 Aufnehmer: für die y-Komponente des lokalen magnetischen Wechselfeldes
- 86 Aufnehmer: für die z-Komponente des lokalen magnetischen Wechselfeldes
- 87 Aufnehmer: für das auf den Körper eines Menschen durch kapazitive Induzierung einwirkende elektrische Wechselfeld
- 88 Aufnehmer: für das im Körper, insbesondere im Gehirn produzierte Summenfeld (EEG) eines Menschen.

Die Ausgangssignale der Aufnehmer werden über einen Multiplexer 89 durch zeitliche Verschachtelung zusammengefaßt. Der Multiplexer 89 speist drei parallele Zweige, die eingangseitig jeweils ein Tiefpaßfilter zur Unterdrückung der bei der Abtastung entstehenden, unerwünschten höheren Frequenzanteile haben, die bei ungünstigen Phasenbedingungen auch zu einer Selbsterregung der gesamten Einrichtung führen könnten. Angenommen ist eine Schwerpunktfrequenz des zu kompensierenden lokalen Wechselfeldes bei etwa 50 Hz. Auch soll eine demgegenüber niedrigere Schwingung im Sinne der anhand der Figur 2 erläuterten Interferenzschwingung mit einer Frequenz unterhalb von 10 Hz vorliegen und das stationäre magnetische Feld (Erdmagnetfeld und gegebenenfalls vorhandene weitere magnetische Gleichfelder) berücksichtigt werden. Für das 50 Hz-Feld ist ein Tracking-Filter 813 von der anhand der Figur 5 erläuterten Art vorgesehen, dem ein Tiefpaß 810 mit einer Grenzfrequenz von 50 Hz vorgeschaltet ist. Für das demgegenüber in der Frequenz niedrigere Wechselfeld ist ein entsprechendes Tracking-Filter 814 mit einem vorgeschalteten Tiefpaßfilter 811 vorgesehen, dessen Grenzfrequenz bei etwa 10 Hertz liegt. Für das dem stationären magnetischen Feld entsprechende Signal liegt im dritten der parallelen Zweige ein Tiefpaß mit einer Grenzfrequenz um etwa 1 Hz. Die PLL-Schaltungen der Tracking-Filter 813 und 814 ermitteln in serieller Verarbeitung die Amplitude und die Frequenz der jeweiligen Felder, und zwar jeweils nach den drei Feldkomponenten des kartesischen Kordinatensystems (x, y, z) zeitlich getrennt. Die in den Ausgängen der Tracking-Filter 813 und 814 abnehmbaren Ergebnisse werden zusammen mit den Feldkompenenten des magnetischen Gleichfeldes, die über den Tiefpaß 812 geführt werden, den Eingängen des Multiplexers 815 zugeführt, der sie zur Auswertung in serieller Form einem Mikrocontroller 816 zuführt.

Die Schwingungserzeugung erfolgt beim Ausführungsbeispiel analog zur Figur 5 wieder durch einen mit einer Steuerspannung in der Frequenz einstellbaren VCO 817. Die Einstellung der Frequenz des VCO 817 erfolgt durch den Mikrocontroller, der diesbezüglich die im von 813 und 814 über den Multiplexer 815 zugeführten Signale auswertet. Vom VCO 817 werden über regelbare Dämpfungsglieder 818, 819 und 820 die Sendespulen x, y, z gespeist, die im begrenzten Raumbereich das kompensierende und/oder interferierende Gegenfeld aufbauen. Die Einstellung der Dämpfungsglieder und damit der Amplitudenwerte der einzelnen Feldkomponentan des Gegenfeldes erfolgt wiederum durch den Mikrocontroller 816 auf der Basis der ihm über 813, 814 und 815 zugeführten Signale. Die Steuerleitung für den VCO und die drei Dämpfungsglieder sind gestrichelt angedeutet.

Da dem Mikrocontroller außerdem die Feldkomponenten des stationären Magnetfeldes und die EEG-Signale zugeführt werden, können in diesem auch möglicherweise auftretende Resonanzen von Zyklotron-Typ und vom NMR-Typ ausgewertet und in einer Anzeigevorrichtung 823, wie einem Leuchtdioden-Array erkennbar gemacht werden. Ebenso ist vom Mikroprozessor 816 eine Frequenzanzeige 821 und eine Amplitudenanzeige 822 für das lokale Wechselfeld und die anderen Wechselfelder, wie das Gegenfeld und das Feld einer Interferenzschwingung ableitbar.

## Patentansprüche

1. Einrichtung zur Beeinflussung von lokalen elektrischen und magnetischen Wechselfeldern niedriger Frequenz, die auf eine in einem begrenzten Raumbereich befindliche leitfähige Struktur, wie die organische Substanz eines Lebewesens, einwirken, bei der eine Aufnahmevorrichtung vorgesehen ist, die in dem begrenzten Raumbereich die Felder in Form von nach einem Koordinatensystem, wie einem kartesischen System, orientierten Feldkomponenten aufnimmt, aus denen der Kompensation der Wechselfelder dienende Felder abgeleitet werden,
**dadurch gekennzeichnet,**
daß an die Aufnahmevorrichtung eine Meßvorrichtung angeschaltet ist, die der Bestimmung der von der Aufnahmevorrichtung aufgenommenen Feldkomponenten nach Amplitude, Frequenz und Orientierung dient, daß an die Meßvorrichtung über ein Steuerungsglied ein Sender angeschaltet ist, der über eine Vorrichtung in dem begrenzten Raumbereich ein Gegenfeld erzeugt, daß das Steuerungsglied, der Sender und die das Gegenfeld erzeugende Vorrichtung in Abhängigkeit von den Meßwerten der Meßvorrichtung so in der Frequenz, in der Stärke und der Koordinatenorientierung des Gegenfeldes einstellbar sind, daß das in dem begrenzten Raumbereich erzeugte Gegenfeld durch Interferenz mit den lokalen Feldern deren Wirkung auf die leitfähige Struktur zumindest näherungsweise kompensiert, und daß der Sender entweder über die an ihn angeschaltete Vorrichtung außer dem die lokalen Felder kompensierenden, in der Schwerpunktfrequenz übereinstimmenden Gegenfeld ein weiteres Schwingungsfeld mit demgegenüber niedrigerer Frequenz, insbesondere mit einem Frequenzwert zwischen etwa 1 Hz und etwa 8 Hz oder etwa 10 Hz und etwa 30 Hz, in dem begrenzten Raumbereich erzeugt, oder in seiner Frequenz gegenüber der Schwerpunktsfrequenz der lokalen Felder um einen solchen Frequenzwert verschoben ist, daß in dem begrenzten Raumbereich eine Interferenzschwingung relativ niedriger Frequenz, bezogen auf die Schwerpunktsfrequenz der lokalen Felder, insbesondere mit einem Frequenzwert zwischen etwa 1 Hz und etwa 8 Hz oder etwa 10 Hz und etwa 30 Hz entsteht.

2. Einrichtung nach Anspruch 1,
**dadurch gekennzeichnet,**
daß die Einrichtung mit einem die Auswertung auf die Feldkomponenten der lokalen Felder begrenzenden Frequenzfilter, insbesondere einem Tiefpaß, versehen ist.

3. Einrichtung nach Anspruch 1 oder 2,
**dadurch gekennzeichnet,**
daß eine Schleifenschaltung vorgesehen ist, die in bestimmten zeitlichen Abständen für eine kurze Zeitspanne die Einstellung des Steuerungsgliedes nach vorgegebenen Frequenzkriterien überprüft und bei Erfüllung derselben jeweils den Sender von der Aufnahmevorrichtung entkoppelt.

4. Einrichtung nach einem der vorhergehenden Ansprüche,
**dadurch gekennzeichnet,**
daß eine zweite Aufnahmevorrichtung vorgesehen ist, die der Bestimmung des in dem begrenzten Raumbereich herrschenden magnetischen Gleichfeldes, insbesondere des Erdmagnetfeldes nach Stärke und Koordinatenausrichtung vorgesehen ist, und daß eine Vorrichtung, der die Signale der ersten und zweiten Aufnahmevorrichtung zugeführt werden, vorgesehen ist, die der Bestimmung von in der leitfähigen Struktur auftretenden Resonanzen dient.

5. Einrichtung nach einem der vorhergehenden Ansprüche,
**dadurch gekennzeichnet,**
daß die Frequenz der Interferenzschwingung und die Sendeenergie in Abhängigkeit von dem im begrenzten Raumbereich gegebenen magnetischen Gleichfeld, wie dem Erdmagnetfeld, so hoch einstellbar sind, daß die Interferenzschwingung im Zusammenwirken mit dem magnetischen Gleichfeld die Bedingungen für eine Zyklotronresonanz mit Ionen, insbesondere Kalzium-Ionen (Ca⁺⁺), von Kalium-Ionen (K⁺) oder von Natrium-Ionen (Na⁺), oder einer nuclear-magnetic-resonance (NMR) einer organischen Substanz erfüllt.

6. Einrichtung nach einem der vorhergehenden Ansprüche, mit einer Feldkomponenten kartesisch erfassenden Aufnahmevorrichtung,
**dadurch gekennzeichnet,**
daß für jede Koordinatenrichtung je ein Aufnehmer für die elektrische und ein Aufnehmer für die magnetische Feldkomponente vorgesehen ist und diese Aufnehmer an die Eingänge eines Multiplexers angeschaltet sind, und daß der Multiplexer-Ausgang, vorzugsweise über einen den auszuwertenden Frequenzbereich aussiebenden Filterzweig, wie einen Tiefpaß, mit der Meßvorrichtung verbunden ist.

7. Einrichtung nach Anspruch 6,
**dadurch gekennzeichnet,**
daß an den Multiplexer-Ausgang mehrere Filterzweige für unterschiedliche Frequenzbereiche angeschaltet sind und an jeden dieser Filterzweige eine Meßvorrichtung angeschaltet ist.

8. Einrichtung nach Anspruch 6 oder 7,
**dadurch gekennzeichnet,**
daß die Meßeinrichtung aus einer Tracking-Filter-Schaltung in PLL-Ausführung besteht, die einen Schaltungsteil für die Abgabe eines der zu ermittelnden Frequenz entsprechenden Signals und einen Schaltungsteil zur Abgabe eines der zu ermittelnden Amplitude entsprechenden Signals umfaßt.

9. Einrichtung nach einem der Ansprüche 6 bis 8,
**dadurch gekennzeichnet,**
daß am Multiplexer ein weiterer Eingang für an der organischen Substanz kapazitiv influenzierte elektrische Felder vorgesehen ist.

10. Einrichtung nach einem der Ansprüche 6 bis 9,
**dadurch gekennzeichnet,**
daß am Multiplexer ein weiterer Eingang für in der organischen Substanz entstehende Felder vorgesehen ist.

11. Einrichtung nach einem der Ansprüche 6 bis 10,
**dadurch gekennzeichnet,**
daß die Ausgänge der einzelnen Meßeinrichtungen mit entsprechenden Eingängen eines weiteren Multiplexers verbunden sind, an dessen Ausgang ein, vorzugsweise digital arbeitender, Mikrokontroller angeschaltet ist, mit dem Einstellorgane für die einzelnen Feldkomponenten des in dem begrenzten Raumbereich von der Einrichtung erzeugten Feldes gesteuert werden.

12. Einrichtung nach einem der vorhergehenden Ansprüche,
**dadurch gekennzeichnet,**
daß als Aufnahmevorrichtung für kapazitiv auf die leitfähige Struktur einwirkende oder in dieser entstehende elektrische Felder an die Struktur anlegbare galvanische Elektroden vorgesehen sind.

13. Einrichtung nach einem der vorhergehenden Ansprüche,
**dadurch gekennzeichnet,**
daß als Aufnahmevorrichtung für induktiv auf die leitfähige Struktur einwirkende magnetische Felder Spulen vorgesehen sind.

14. Einrichtung nach einem der vorhergehenden Ansprüche,
**dadurch gekennzeichnet,**
daß als Aufnahmevorrichtung für das in dem begrenzten Raumbereich einwirkende Erdmagnetfeld ein Magnetoflux-Aufnehmer (dreidimensional) oder ein, vorzugsweise temperaturkompensierter, Hallgenerator vorgesehen ist.

## Claims

1. A device for controlling localised low-frequency electric and magnetic alternating fields which have an effect on a conductive structure located within a delimited volume of space, such as the organic substance of a living organism, which has a pick-up device which picks up the fields within a delimited volume of space in the form of field components oriented according to a system of co-ordinates such as a Cartesian system, from which are then derived the fields used for compensating the alternating fields,
**characterised in that,**
a measurement device is connected to the pick-up device, which is used for determining amplitude, frequency and orientation of the field components picked up by the pick-up device, in that a transmitter which uses a device to generate an opposite field in the delimited volume of space is connected to the measurement device by means of a control element, in that the frequency, the strength and the opposite field co-ordinate orientation of the control element, the transmitter and the device which generates the opposite field can be adjusted in accordance with the measured values from the measurement device so that the opposite field generated in the delimited volume of space interferes with the localised fields in such a way as almost approximately to compensate for their effects on the conductive structure, and in that the transmitter either uses the device connected to it to generate a further oscillation field in the delimited volume of space with a frequency lower than that of the opposite field used to compensate the localised fields with the same concentration frequency, in particular an oscillation field with a frequency value between approximately 1 Hz and approximately 8 Hz or approximately 10 Hz and approximately 30 Hz, or that the frequency of the transmitter is displaced in relation to the concentration frequency of the localised fields by such a frequency value as to cause an interference oscillation to be generated within the delimited volume of space, with the interference oscillation being of a relatively low frequency in relation to the concentration frequency of the localised fields, in particular, with a frequency value between approximately 1 Hz and approximately 8 Hz or approximately 10 Hz and approximately 30 Hz.

2. The device in accordance with Claim 1,
**characterised in that,**
the device is equipped with a frequency filter which restricts evaluation to the field components of the localised fields, in particular a lowpass.

3. The device in accordance with Claim 1 or 2,
**characterised in that,**
a loop circuit is provided, which monitors the adjustment of the control element briefly at regular intervals in accordance with specified frequency criteria, and which, given compliance with the aforementioned criteria, decouples the transmitter from the pick-up device in each case.

4. The device in accordance with one of the aforementioned claims,
**characterised in that,**
a second pick-up device is provided in order to determine the constant magnetic field existing within the delimited volume of space, and in particular, the strength and orientation of coordinates of the geomagnetic field; and in that a device is provided to which are sent the signals from the first and second pick-up devices and which is used for determining the resonances occurring within the conductive structure.

5. The device in accordance with one of the aforementioned claims,
**characterised in that,**
the frequency of the interference oscillation and the transmitter power can be set at high enough levels as a function of the given constant magnetic field existing within the delimited volume of space, such as the geomagnetic field, so that the interference oscillation combines with the magnetic constant field to fulfil the conditions for a cyclotron resonance with ions, in particular calcium ions (Ca^{**++**}), potassium ions (K^{**+**}) or sodium ions (Na⁺), or for nuclear magnetic resonance (NMR) of an organic substance.

6. The device in accordance with one of the aforementioned claims, with a pick-up device to pick up the field components using a Cartesian method,
**characterised in that,**
for each co-ordinate direction, one pick-up is provided for the electric field components and one for the magnetic field components and these pick-ups are connected to the inputs of a multiplexer, and in that the multiplexer output is, in a preferred embodiment, connected to the measurement device by means of a filter branch such as a lowpass, which separates out the frequency range to be evaluated.

7. The device in accordance with Claim 6,
**characterised in that,**
several filter branches are connected to the multiplexer output which are used for various frequency ranges and a measurement device is connected to each of these filter branches.

8. The device in accordance with Claim 6 or 7,
**characterised in that,**
the measurement device consists of a PLL-type tracking filter circuit which incorporates one circuit section for outputting a signal corresponding to the frequency to be determined and another circuit section for outputting a signal corresponding to the amplitude to be determined.

9. The device in accordance with one of Claims 6 to 8,
**characterised in that,**
a further input is provided on the multiplexer for electric fields influenced by capacitance on the organic substance.

10. The device in accordance with one of Claims 6 to 9,
**characterised in that,**
another input is provided on the multiplexer for fields arising within the organic substance.

11. The device in accordance with one of Claims 6 to 10,
**characterised in that,**
the outputs from the individual measuring devices are connected to the corresponding inputs on a further multiplexer, the output of which is connected to a microcontroller, which, in a preferred embodiment, functions digitally and which gives control over the adjusting elements for the individual field components of the field generated by the device in the delimited volume of space.

12. The device in accordance with one of the aforementioned claims,
**characterised in that,**
galvanic electrodes are provided, which can be placed against the structure as a pick-up device for electric fields influencing the conductive structure by capacitance or for fields arising within the conductive structure.

13. The device in accordance with one of the aforementioned claims,
**characterised in that,**
coils are provided as a pick-up device for magnetic fields which influence the conductive structure by inductance.

14. The device in accordance with one of the aforementioned claims,
**characterised in that,**
a magnetic flux meter (three dimensional) or a Hall generator which, in a preferred embodiment, is temperature compensating, is provided as a pick-up device for the geomagnetic field which exerts an influence within the delimited volume of space.

## Revendications

1. Dispositif permettant d'influer sur des champs alternatifs électriques et magnétiques locaux de basse fréquence, agissant sur une structure conductrice se trouvant dans un endroit délimité, telle qu'une substance organique d'un être vivant, le dispositif comprenant un organe de recevant, dans l'endroit délimité, les champs sous la forme de composants de champ orientés selon un système de coordonnés comme un système cartésien, d'où sont dérivés des champs servant à la compensation des champs alternatifs,
caractérisé en ce que,
sur l'organe de réception il est branché un dispositif de mesure servant à identifier l'amplitude, la fréquence et l'orientation des composants de champ reçus par l'organe de réception, que sur le dispositif de mesure il est branché un émetteur par l'intermédiaire d'un membre de commande qui, moyennant un dispositif, engendre dans l'endroit délimité un contre-champ, que, en dépendance des valeurs retenues par le dispositif de mesure, il est possible de régler la fréquence, l'intensité et les coordonnés d'orientation du contre-champ du membre de commande, de l'émetteur et du dispositif engendrant le contre-champ, de sorte que par interférence avec les champs locaux, le contre-champ engendré dans l'endroit délimité compense du moins approximativement leur effet sur la structure conductrice, et que l'émetteur engendre par l'intermédiaire du dispositif branché sur celui-ci, non seulement un contre-champ compensant les champs locaux et coïncidant dans la fréquence principale, mais de plus un autre champ d'oscillations dont la fréquence est plus basse, notamment avec une fréquence comprise entre 1 Hz et env. 8 Hz ou entre env. 10 Hz et env. 30 Hz, ou bien que sa fréquence est décalé par rapport à la fréquence principale des champs locaux de sorte que dans l'endroit délimité, il est engendré une oscillation d'interférence d'une fréquence relativement basse, référée à la fréquence principale des champs locaux, notamment avec une fréquence comprise entre 1 Hz et env. 8 Hz ou entre env. 10 Hz et env. 30 Hz.

2. Dispositif d'après la revendication 1,
caractérisé en ce que
le dispositif est équipé d'un filtre de fréquence limitant les répercussions sur les composants de champ des champs locaux, en particulier un filtre passe-bas.

3. Dispositif d'après la revendication 1 ou 2,
caractérisé en ce qu'il est prévu une connexion en boucle qui, à périodicités régulières, contrôle pendant une brève période le réglage du membre de commande en vue de critères de fréquence donnés et qui, ceux-ci sont remplis, découple chaque fois le capteur du dispositif de réception.

4. Dispositif d'après une des revendications précédentes,
caractérisé en ce
qu'il est prévu un deuxième dispositif de réception pour déterminer l'intensité et des coordonnés d'orientation du champ magnétique équidirectionnel régnant dans l'endroit délimité, notamment le champ magnétique terrestre, et qu'il est prévu un autre dispositif recevant les signaux du premier et du deuxième dispositif de réception et identifiant les résonances apparaissant dans la structure conductrice.

5. Dispositif d'après une des revendications précédentes,
caractérisé en ce que
le niveau de la fréquence de l'oscillation d'interférence ainsi que de l'énergie d'émission se laisse régler en dépendance du champ magnétique équidirectionnel régnant dans l'endroit délimité, comme le champ magnétique terrestre, de sorte qu'en collaboration avec le champ magnétique équidirectionnel l'oscillation d'interférence remplit les conditions pour une résonance gyromagnétique avec des ions, en particulier des ions de calcium (Ca⁺⁺), des ions de potassium (K⁺) ou des ions de sodium (Na⁺) ou bien d'une résonance magnétique nucléaire (NMR) d'une substance organique.

6. Dispositif d'après une des revendications précédentes, avec dispositif de réception pour la saisie cartésienne des composants de champ,
caractérisé en ce que
pour chaque coordonné, il est prévu respectivement un capteur pour le composant de champ électrique et un capteur pour le composant de champ magnétique et que ces capteurs sont branchés sur les entrées d'un multiplexeur, et que la sortie du multiplexeur est raccordée au dispositif de mesure disposé de préférence dans une branche de filtrage, tel qu'un filtre passe-bas, filtrant la gamme de fréquences à évaluer.

7. Dispositif d'après la revendication 6,
caractérisé en ce que
sur la sortie du multiplexeur, il est branché plusieurs branches de filtrage pour les plages de fréquences différentes et que sur chacune de ces branches de filtrage, il est raccordé un dispositif de mesure.

8. Dispositif d'après la revendication 6 ou 7,
caractérisé en ce que
le dispositif de mesure constitue une connexion à filtre suiveur en circuit PLL comportant un circuit de commande pour l'émission d'un signal correspondant à la fréquence à déterminer, ainsi qu'un circuit de commande pour l'émission d'un signal correspondant à l'amplitude à déterminer.

9. Dispositif d'après une ou plusieurs des revendications 6 à 8,
caractérisé en ce que
sur le multiplexeur, il est prévu une autre entrée pour des champs électriques influés de manière capacitive sur la substance organique.

10. Dispositif d'après une ou plusieurs des revendications 6 à 9,
caractérisé en ce que
sur le multiplexeur, il est prévu une autre entrée pour des champs générés dans la substance organique.

11. Dispositif d'après une ou plusieurs des revendications 6 à 10,
caractérisé en ce que
les sorties des divers dispositifs de mesure sont raccordées aux entrées correspondantes d'un autre multiplexeur dont la sortie est raccordée de sa part à un micro-contrôleur de préférence digital qui permet de commander des organes de réglage pour les divers composants du champ généré par le dispositif dans l'endroit délimité.

12. Dispositif d'après une des revendications précédentes,
caractérisé en ce que,
en tant que dispositif de réception pour les champs électriques agissant de manière capacitive sur la structure conductrice ou naissant dans celle-ci, il est prévu des électrodes galvaniques se laissant appliquer sur la structure.

13. Dispositif d'après une des revendications précédentes,
caractérisé en ce que,
en tant que dispositif de réception pour les champs magnétiques agissant de manière inductive sur la structure conductrice, il est prévu des bobines.

14. Dispositif d'après une des revendications précédentes,
caractérisé en ce que,
en tant que dispositif de réception pour le champ magnétique terrestre agissant sur l'endroit délimité, il est prévu un capteur magnétoflux (tridimensionnel) ou un générateur de Hall de préférence à compensation de température.
